# EUROPEAN PATENT APPLICATION

(11) **EP 2 698 429 A1**
(43) Date of publication of application: **19.02.2014**
(21) Application number: 12180788.7
(22) Date of filing: 17.08.2012
(51) Int. Cl.: C12N 5/00

(54) **Support for the culture of adherent cells and method for their detachment**

(71) Applicant: Centre National de la Recherche Scientifique (CNRS), 75794 Paris Cedex 16 (FR); GENETHON, 91002 Evry Cedex (FR)
(72) Inventor: Brun-Graeppi, Amanda, 92370 Chaville (FR); Scherman, Daniel, 75012 Paris (FR); Bessodes, Michel, 94800 Villejuif (FR); Richard, Cyrille, 78180 Montigny le Bx (FR); Merten, Otto, 91002 Evry cedex (FR)
(74) Representative: Peaucelle, Chantal

(57) **Abstract**

The invention relates to cell culture support comprising a functionalized xyloglucan hydrogel-based film, such as produced by
. removal of at least 35%, preferably from 35-45%, of the galactose groups of the xyloglucan residue,
. activation of part of the remaining free -OH groups of the sugar residues of the modified xyloglucan (galactose, xylose)
for grafting a bioadhesive peptide as ligand or containing the specific motifs of ligand specific for the attachment site of cell surface proteins.

## Description

The invention relates to a new support for the culture of adherent cells and a method for their detachment. It more particularly relates to a thermoresponsive support.

The optimization of cell amplification, particularly stem cells, protocols is a key point in biotechnology. In this context, cell detachment is an important step.

Enzymatic detachment represents the most widespread method for adherent cell dissociation. It consists in the use of enzymes such as collagenase, dispase, or trypsin [Merten 2010]. Although enzymatic methods readily enable large-scale expansion, they may result in cell damage. Indeed, this approach was observed to be quite inadequate for detaching some cells, such as microglia, because this treatment altered their metabolism and cellular responses.

An alternative for the enzymatic method is the use of mechanical ones. They avoid enzymes and seem to maintain genetic stability. In addition to that, mechanical expansion allows selective transfer of exclusively undifferentiated colonies, considering human embryonic stem cells (HESC). However, the comparatively laborious and time-consuming process of manual colony dissection limits the practical use of mechanical passaging in bulk cell culture [Joannidesa et al. 2006]. Therefore, it would be useful to have innovative approaches to overcome the presented drawbacks related to cell passaging.

Developing culture surfaces of stimuli-responsive polymers represent an interesting possibility. Stimuli-responsive polymers are defined as polymers that undergo relatively large and abrupt, physical or chemical changes in response to external stimuli. Such stimuli could be classified as either chemical or physical, such as temperature. In culture substrates based on stimuli-responsive polymers, cell detachment can take place by adjusting such physical or chemical parameters, instead of using mechanical or enzymatic methods.

In this context, the inventors have evaluated xyloglucan, a major hemicellulose component of primary walls of many higher plants, for temperature-assisted cell detachment.

This polysaccharide has a backbone consisting of glucose residues, with side groups of xylose attached through glycosidic linkages. Some xylose residues are additionally substituted with galactose or the disaccharide galactose and fucose.

Xyloglucan can be rendered thermally responsive by using fungal β-galactosidase to remove more than 35% of the galactose residues [Shirakawa et al. 1998]. The thermo-sensitive property of xyloglucan has been mainly exploited in pharmaceutical technology in the development of oral and rectal delivery of drugs and for intraperitoneal injections. This modified xyloglucan is characterized by sol-gel transition at a certain temperature in function of the percentage of residual galactose residues. The more groups are removed, the lower is the transient temperature at which a xyloglucan solution passes from a solution to a gel state or vice versa.

Its use as a synthetic extracellular matrix has already been proposed, but without any modification. However, in such a case, the interest in the polymer lied in its galactose moieties for hepatocyte attachment [Seo et al. 2004].

The inventors have found that cell culture surfaces of great interest could be elaborated by chemically modifying xyloglucans with peptides comprising the cell attachment site of surface proteins.

An object of the invention is to provide a support for cell culture, particularly stem cells

Another object is to provide a method for the detachment of the adherent cells from said culture surfaces.

According to the invention, the cell culture support comprises a functionalized xyloglucan hydrogel-based film, such as produced by
- removal of at least 35%, preferably from 35-45%, of the galactose groups of the xyloglucan residue,
- activation of part of the remaining free - OH groups of the sugar residues of the modified xyloglucan (galactose, xylose)
for grafting a bioadhesive peptide as ligand or containing the motifs specific for cell surface matrix receptors/cell surface proteins. The partial galactose removal step is carried out by reacting the xyloglucan with β-galactosidase. Advantageously the reaction is carried out during 20h-30h, preferably during about 24h at a temperature above 45°C, particularly of 50-55°C.

The activation step comprises reacting at least some of the remaining groups of the partially degalactosylated xyloglucan with an agent such as 4-nitrophenyl chloroformate. The reaction is advantageously carried out at room temperature.

After said activation reaction, xyloglycan is reacted with a peptide containing the cell attachment site of surface proteins, particularly a sequence part of an integrin-ligand. The reaction is also advantageously carried out at room temperature.

A preferred group of peptides contain the sequence RGD. Adhesion proteins containing said sequence comprise fibronectin, vitronectin, fibrinogen, von Willebrand factor, thrombospondin, laminin, entactin, tenascin, osteopontin, bone sialoprotein.

A particularly preferred peptide comprises a cyclic peptide DfKRG presenting the RGD motif.

Another preferred peptide containing the sequence RGD is CGGNGEPRGDTYRAY.

Other preferred peptides are selected in the group comprising KKQRFRHRNRKG from vitronectin, CDPGYIGSR, IKVAV, RNIAEIIKDI, YFQRYLI, PDSRG LNIVSVNGRHX, and DNRIRLQAKXX from laminin, DGEA and GVKGDKGNPGWPGAE peptides from collagen, VAPG sequence of elastin, SIGFRGDGQTC from entactin and FHRRIKA from bone sialoprotein.

The support coated on the cell culture surface is incubated with the culture medium for conditioning for 24h.

To obtain cell culture supports satisfactorily resisting to culture conditions, hydrogels of more than 2% of xyloglucan residue, advantageously of about 4% are used.

The rheological properties may be modified through the variation of removal rate of galactose simply by changing the enzyme reaction time.

This allows for example to obtain a range of products in the same batch by taking samples at different reaction times. Also the range of products that can be easily obtained with different operating temperatures of sol-gel transition is an asset that can be useful to counteract the effect of grafting of bioactive molecules.

According to an embodiment of the invention, an hydrogel containing 3% at least, preferably around 5%, with a partially degalactosylated xyloglucan grafted with a bioadhesive peptide is used.

In another embodiment a mixture of said hydrogel at 3% at least, preferably around 5%, with degalactosylated, non grafted non activated xyloglucan is used at about 2-4%, preferably 3%. An advantageous mixture comprises the grafted xyloglucane and non grafted xyloglucane in a ratio of 1:1vol/vol.

In another embodiment of the invention, to enhance the interaction between the peptide and the cell, a spacer is used. An appropriate spacer is PEG with MW of about 1100 daltons.

In another embodiment of the invention, the preferred use of the partially degalactosylated hydrogel is in form of a film such as obtained by drying.

The culture cell films are prepared by drying the hydrogel. Preferred conditions for drying the hydrogel comprise heating the hydrogel at above 40°C, particularly between 50-70°C, under vacuum, during about at least 1H, preferably between 1-3h, particularly during 2h.

Advantageously, the above-defined culture film is temperature-responsive and temperature-assisted detachment can be carried out.

The invention thus also relates to a method for detachment of adherent cells from a support, temperature-assisted. The preferred cell culture temperature for mammalian cells is about 37°C.

The detachment may be obtained at room temperature.

Preferably, the culture medium is changed for fresh one and incubated.

A reduction of the temperature is applied, for example by addition of fresh culture medium and incubation.

The temperature of the culture medium is preferably lower than 10°C, preferably between 5 and 3°C.

Advantageously, the culture support of the invention is animal-free, thermosensitive, sterilizable, able to promote attachment of cells while allowing their detachment by reduced temperature. This approach eliminates the need to use proteases which enhances the security of the method, by avoiding any eventual contamination of the cells by adventitious agents. The absence of use of protease is also important to maintain the intercellular junctions. This may be helpful if cell sheets are desired. In this case, adherent cells may be cultured until confluence and eventually detached as a monolayer.

For sterilization, the solution of degalactosylated xyloglucan grafted with the bioadhesive peptides are autoclaved or irradiated in frozen state before use as coating culture supports. With fragile peptides, particularly linear peptides, irradiation of frozen culture supports is preferred.

According to another advantage resulting from the use of the above films, it is possible to obtain hydrogels with a broad range of products in the same batch.

Other characteristics and advantages of the invention are given in the following examples.

In said examples, it is referred to Figures 1-9 which represent, respectively:
Figure 1: Grafting RGD containing peptide (cyclic DfKRG) to xyloglucan degalactosylated for 24h (T24). Step A: Activation of T24 hydroxyl groups by 4-nitrophenyl chloroformiate. Step B: Functionalization of activated T24 groups by reaction with DfKRG.
Figure 2: Temperature dependence of G' and G" for autoclaved T0 (non-galactosylated xyloglucan), T24 (xyloglucan degalactosylated for 24h) and T24-DfKRG (xyloglucan degalactosylated for 24h and grafted with cyclic DfKRG) in PBS at 2%.
Figure 3: Attachment of A375 cells on three types of support. A: uncoated polystyrene plate (control). B: T24 (xyloglucan degalactosylated for 24h) coated polystyrene plate. C: T24-RGD (xyloglucan degalactosylated for 24h and grafted with cyclic DfKRG) coated polystyrene plate. Images of wells two days after cell seeding at 100 X magnification (20.000 seeded cells per well - 24-well plate).
Figure 4: Temperature-assisted cell detachment from T24 (xyloglucan degalactosylated for 24h) and T24-RGD (xyloglucan degalactosylated for 24h and grafted with cyclic DfKRG) coated wells after 7 days of culture (20.000 A375 cells seeded per well- 24-well plate).
Figure 5: Cell detachment kinetics from T24-RGD (xyloglucan degalactosylated for 24h and grafted with cyclic DfKRG) films after 7 days of culture (at three different temperatures) (the amount of cells detached by trypsinization was considered as 100% cell detachment).

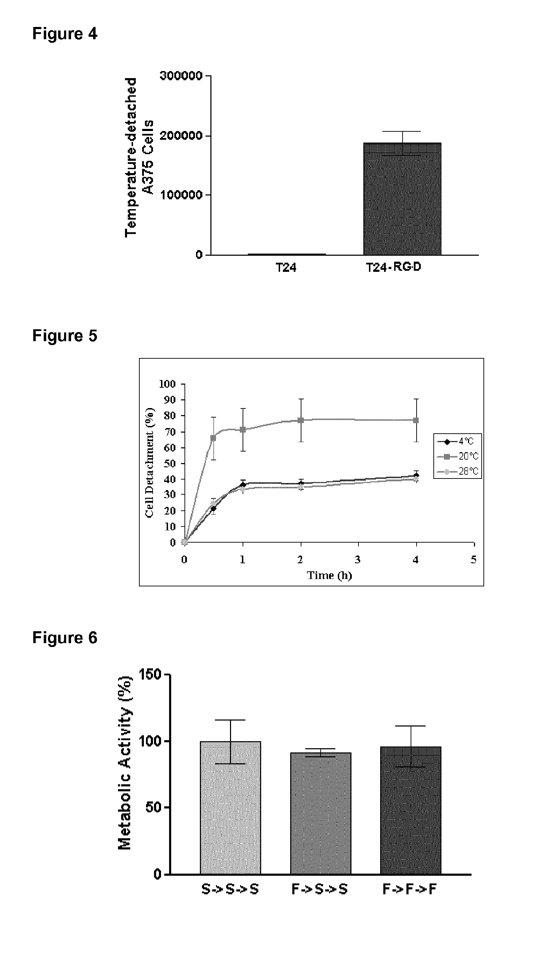

Figure 6: Metabolic activity of A375 cells after a 3-week culture (assessed by MTT). S→S→S stands for cells that underwent three passages on standard uncoated wells using trypsinization for cell detachment (control). F→F→F stands for cells that underwent three passages on T24-RGD (xyloglucan degalactosylated for 24h and grafted with cyclic DfKRG) films using the temperature-assisted method for cell detachment. F→S→S stands for cells that underwent one passage on T24-RGD films using the temperature-assisted method for cell detachment and two passages on standard uncoated wells using trypsinization for cell detachment. Data are normalized to the control level of cell metabolic activity.
Figure 7: Metabolic activity of A375 cells plated on standard uncoated wells (control) or on T24-RGD (xyloglucan degalactosylated for 24h and grafted with cyclic DfKRG) coated culture wells and submitted to the trypsinisation (control) or temperature-assisted method (T24-RGD) for cell detachment (20°C 1h) (assessed by MTT test). Data are normalized to the control level of cell metabolic activity.
Figure 8: Assessment of the effect of different concentration of T24-RGD (xyloglucan degalactosylated for 24h and grafted with cyclic DfKRG) on the metabolic activity of A375 cells (assessed by MTT test). Cells were incubated with 1, 5 and 10 mg/mL of T24-RGD. Data are normalized to the control level of cell metabolic activity.
Figure 9: Cell proliferation on uncoated standard wells during three weeks after a first passage from T24-RGD coated wells. Control concerns cell proliferation exclusively on uncoated standard wells. Cells were passaged once a week (20.000 cells per well - 24-well plate).

### Materials and Methods

### β-galactosidase reaction

The production of thermoresponsive xyloglucan at a galactose removal ratio (GRR) of 40% was carried out by reacting 1 g of xyloglucan from *Tamarindus indica* (Megazyme International, Ireland) in 50 mL of aqueous medium with 31.35 mg of β-galactosidase from Aspergillus oryzae (Sigma, USA) at 11.8 units/mg, as reported previously [Andriola Silva Brun-Graeppi et al. 2010]. Briefly, the reaction was carried at 53°C during 24 hours. The sample was then heated at 100°C for 20 min to inactivate the enzyme. The partially-degalactosylated xyloglucan was precipitated from this solution by addition of ethanol. After three cycles of redispersion in water/precipitation in ethanol for purification, the product was dried at 60°C during two days. This sample is thereafter identified as T24. The supernatant obtained after purification was analyzed to confirm the GRR using an enzymatic kit, [Lactose / D-Galactose (Rapid) Assay Kit - Megazyme International Ireland Ltd. (Ireland)]. The GRR ratio was determined as (Released galactose residues x 100)/total galactose residues.

The amount of total galactose residues was measured after total hydrolysis by heating the polymer with 2N sulfuric acid at 100°C for 3 hours [Shirakawa et al. 1998].

### RGD grafting on partially degalactosylated xyloglucan

After the enzymatic reaction, some remaining hydroxyl groups of the polymer were reacted with p-nitrophenyl chlorocarbonate (NPC) [Zhang et al. 2007] to achieve an activated material able to react with the RGD peptide.

In this strategy, 600 mg of T24 were solubilized in 40 mL of dimethylsulfoxide (DMSO, Sigma, Germany) at 50°C during 3 hours. Then, 2.4 g of 4-nitrophenyl chloroformate (Sigma, Germany) were added, followed by 40 mL of pyridine (SDS, France) and 240 mg of 4-dimethylaminopyridine (DMAP, Fluka, Switzerland). After overnight stirring at room-temperature, the reaction product was precipitated with ethanol (50 mL) and recovered. This process was repeated three times. The degree of substitution was determined spectrophotometrically by measuring, at 402 nm (ε = 18400 cm⁻¹ M⁻¹) with a UV-Visible Cary 100 spectrophotometer (Varian, USA), the amount of p-nitrophenylate released after reaction with NaOH (2N) [Marre et al. 1995]. We used 1 mL of NaOH (2N) per mg of product.

After the activation reaction, xyloglucan was reacted overnight with the RGD peptide at room temperature in an anhydrous basic medium. The purified activated T24 was stirred in 75 mL of DMSO at 50°C during 3 hours. After solubilization, 120 mg of DfKRG cyclic peptide (American Peptide, USA), 1.7 mL of triethylamine (SDS, France) and 1.47 g of DMAP (Fluka, Switzerland) were added. The reaction was then allowed to proceed overnight under stirring at room temperature and was followed by the inactivation of unreacted nitrophenylcarbonate groups with 600 mL of triethylammonium acetate buffer at 60°C during 4 days.

Amino acid analysis, using an Aminotac JLC500/V equipment (Jeol, Tokyo, Japon), was carried out to evaluate the amount of grafted peptide. For this purpose, 2 mg of T24-RGD was reacted with 6M HCl at 110°C during 24h in order to break peptide bonds. The result was also confirmed by fluorescence spectrophotometry by grafting a FITC-DfKRG peptide (American Peptide, USA) to T24. The purified product was solubilized in water (1 mg/mL) and the fluorescence intensity of this solution was obtained with a Cary Eclipse fluorescence spectrophotometer (Varian, USA). Excitation and emission wavelengths were 488 and 515 nm, respectively. Quantification was performed by means of a calibration curve from standard FITC-DfKRG solutions in water at concentrations ranging from 90 to 2 nmol/ml.

### Rheology

The thermogelation properties of native (T0) and modified xyloglucan T24 and T24-RGD were evaluated by rheology. The oscillatory shear measurements were performed on a stress controlled rheometer RS600 Haake (Germany) equipped with a cone-and-plate geometry (35 mm diameter, 2° angle and gap size of 50 µm). The values of the stress amplitude were checked to ensure that all measurements were conducted within the linear viscoelastic regime, where the dynamic storage modulus (G') and loss modulus (G") are independent of the stress amplitude. The measuring unit was equipped with a temperature unit (Peltier plate) that provided temperature control during analysis. Rheological behaviour of the sol-gel transition was measured by performing temperature sweeps. Temperature dependence of storage (G') and loss (G") moduli were observed by heating the systems from 10°C to 60°C at a rate of 0.5°C/min. The samples were protected by a homemade cover to prevent water evaporation.

Samples were prepared by dissolving 200 mg of T0, T24 or T24RGD in PBS (10 mL) and stirring at 4°C for two days. The obtained solutions were stored at 4°C.

### Cell culture studies

A375 cells (ATCC #CRL-1619), overexpressing the RGD receptor, were cultured on Dulbecco's modified Eagle's medium (DMEM, GIBCO, USA) supplemented with 10% of foetal calf serum (FCS, PAA, Austria) on 75cm² culture flasks (TPP, Switzerland).

Cell studies were performed in 24-well plates (TPP, Switzerland) previously coated with autoclaved T24 and T24-RGD in PBS. Coating was carried out by adding 0.6 mL of each product and drying for 2h at 60°C under vacuum. Coated wells were incubated with culture medium during 24h, and the medium was changed three-times during this period. Several concentrations were tested in order to obtain polymer films resistant to medium incubation. According to the concentration, the obtained film may undergo dissolution even at 37°C after the culture medium is added. We obtained films resisting to culture conditions from T24 hydrogels at 3%. For T24RGD, we used a mixture (1:1 vol/vol) of this grafted hydrogel at 5% with T24 at 3%. Uncoated wells were used as a positive control. Cells were seeded at a density of 20.000 per well and incubated at 37°C under a humidified atmosphere of 5% CO₂ in air. The culture medium changes and the cell passages were carried out twice and once a week, respectively. Cell attachment on T24 and T24-RGD coated wells and uncoated wells was observed by fluorescence microscopy using a Axiovert 135 microscope (Zeiss, Germany) and carboxyfluorescein succinimidyl ester (CFSE, Sigma, Germany) to label cells.

For temperature-assisted cell detachment, the culture medium was replaced by 1 mL of fresh one at 4°C and the plate was incubated for 1h at room temperature. For a kinetics experiment, cell detachment was evaluated at 30, 60, 120 and 240 min at 4, 20 and 28°C. The number of cells detached from the T24 and T24-RGD coated wells was evaluated by hemacytometer cell counting. The number of cells in the uncoated wells was also determined by hemacytometer cell counting after trypsinization. After a three-week culture on T24-RGD coated and uncoated wells, 20.000 cells of each condition were seeded on uncoated wells, incubated overnight and cell metabolic activity was evaluated by spectophotometry using 3-(4,5-dimethylthiazol-2-yl)-2,5-diphenyltetrazolium bromide (MTT, Sigma, Germany) (MTT test). In brief, 5 mL of MTT solution at 5 mg/mL was diluted with 45 mL of culture medium and 1 mL of such solution was added to each well. The plate was incubated at 37°C during 3 hours, the supernatant was carefully aspirated and 1 mL of lysis solution (10g of Triton X-100 and 10 ml of 1 N HCl in anhydrous isopropanol, 100 ml) was added to each well. The optical density was determined at a wavelength of 562 nm.

MTT test was also used to evaluate the effect on the metabolic activity of our temperature-assisted detachment method and effect of cell exposure to different concentrations of T24 and T24-RGD in solution.

### Data analysis

Data are presented as one standard deviation from the mean (n = 3). The Student T-test was performed to determine a significant difference between the experimental and control groups using Prism 3.0 version of GraphPad software (USA). A 95% confidence level was considered significant.

### Example 1: Activation of xyloglucan

Grafting biologically active molecules on polymers is an attractive approach in biotechnology. Our strategy to functionalize T24 with a Arg-Gly-Asp (RGD) sequence involved first an activation step [Marre et al. 1995].

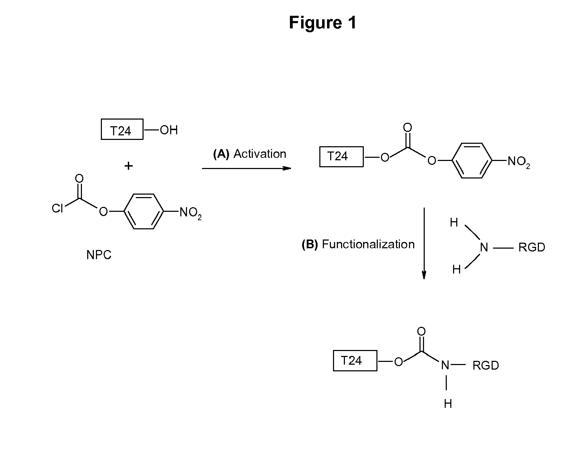

A partial conversion of the primary hydroxyl side groups on T24 polymer chain was carried out using 4-nitrophenyl chloroformate (NPC) (Fig. 1a). After purification, an hydrolysis of a small fraction of the obtained product was done to determine the degree of activation. The presence of an absorption band at 402 nm corresponding to the 4-nitrophenylate confirmed the activation of T24. The concentration of this compound determined spectrometrically indicated a ratio of 6 activated hydroxyl per T24 molecule.

### Example 2: Grafting of RGD peptide

After the activation of T24, the reaction with the RGD peptide (cyclic DfKRG) was studied. The reactive carbonate-containing T24 was reacted overnight with RGD (Fig.1b). After the reaction, a hydrolysis was carried out at pH 8 in triethylammonium acetate buffer in order to remove unreacted activated groups remaining on the polymer. After purification, the efficacy of this step was evaluated by a subsequent treatment of an aliquot with NaOH 0.2 M. No more absorbance band at 402 nm was detected indicating that this procedure successfully removed unreacted activated groups.

The amount of peptide bound per mass of polymer was determined by amino acid analysis. A degree of functionalization of 27 µmol of peptide per g of polymer was obtained. The degree of RGD grafting was also estimated by fluorescence spectrophotometry using a FITC-labelled RGD. With this second method, 14.2 µmol of peptide per gram of grafted polymer was detected, a result in the same order of magnitude as obtained by the amino acid analyzer AminoTac. This corresponds to a grafting ratio of about 5 RGD molecules per T24 molecule.

T24 and T24-RGD were autoclaved in order to obtain sterile samples. As reported in a previously published paper, this method as well as γ-irradiation at 10 kGy (hydrogel irradiation on frozen state) efficiently sterilize xyloglucan-based hydrogels and preserve the rheological properties of the polymer [Andriola Silva Brun-Graeppi et al. 2010].

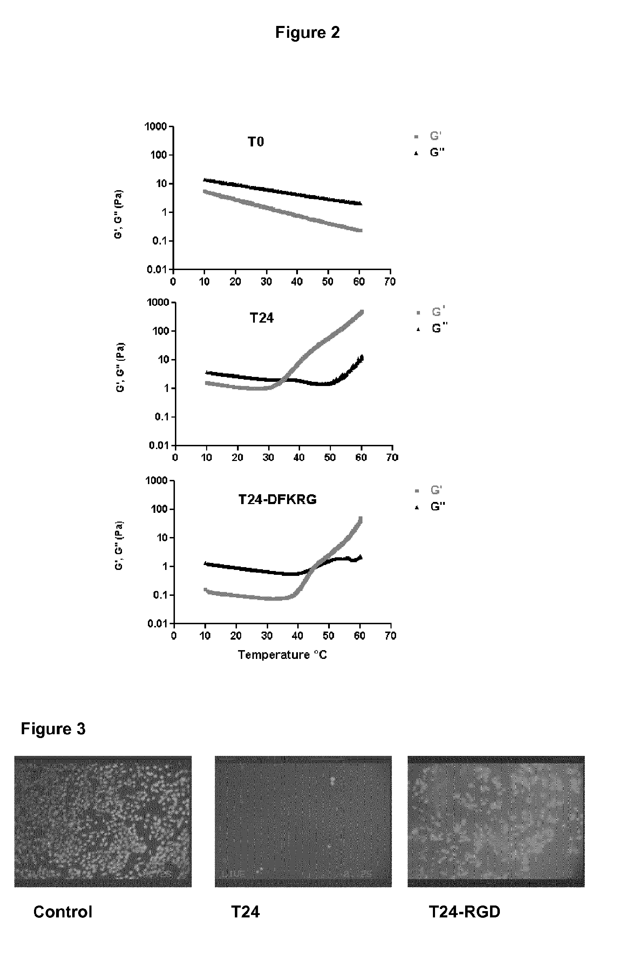

In this study, sol-gel transition evaluation was based on the temperature of G' and G" cross-over. According to the rheological analysis (Fig. 2), the sol-gel transition for T24 and T24-RGD at 2% (w/v) took place at about 37°C and 45°C respectively, for a heating rate of 0.5°C/min. Native unmodified xyloglucan (T0) presents no sol-gel transition. For this sample, G' is always inferior to G" as galactose moieties sterically hinder gelation [Shirakawa et al. 1998].

### Example 3: Cell culture

T24 and T24-RGD were evaluated for cell adhesion, proliferation and metabolic activity assays. A375 cells were chosen as model since RGD peptides can specifically bind with αᵥβ₃ integrin over-expressed on their surface.

The plates were analysed by microscopy 48 h after seeding. A375 cells exhibited higher levels of attachment (Fig. 3) on T24-RGD coated wells when compared to T24-coated ones. The higher level of cell adhesion on T24-RGD was attributed to the functional immobilized peptide. It is likely that the α_{V}β₃ integrins from A375 cells could recognize RGD ligands on the surface, and so attach to the film as a result. However, cell adhesion on uncoated wells was superior than observed for T24-RGD. This may be in part due to the soft surface constituted of a polymer layer. In fact, cell adhesiveness and spreading are enhanced by the rigidity of the substrate. Mechanotransduction across the adhesion site between cell focal adhesion plaques and the extracellular matrix attached to the substrate determines cellular fundamental behaviours including adhesion, spreading, migration, proliferation, differentiation, re-differentiation, metastasis, and apoptosis [Ohya et al. 2005].

At day 7 after seeding, A375 cells on uncoated wells were trypsinized while A375 cells on T24 and T24-RGD coated wells were detached by a 1-hour incubation at room temperature after the addition of 1 mL of fresh medium (4°C). Compared to the number of seeded cells (20 000), the amount of cells detached from T24-coated wells was markedly inferior (1 333 ± 600.9), while an almost 10-fold increase (187 000 ± 11 630) was obtained from T24-RGD coated surfaces (Fig. 4). A significant difference is observed by comparing T24 and T24-RGD groups (P<0.0001). Cells were passaged to T24-RGD coated wells two weeks more. Cells on T24-coated wells could not be passaged as the amount of obtained cells was inferior to 20 000 cells per well. The amount of detached cells by temperature-assisted method increased for T24-RGD in the two following culture weeks (2 following passages): 257 000 ± 18 480 and 330 000 ± 28 870, (20 000 seeded cells per well at each passage). This means that cell proliferation is not reduced after following temperature-assisted detachment steps.

The detachment of cells by reduction of the temperature can be explained by surface swelling and loss of cell tension on hydrated polymer chains. Temperature-dependent swelling of ligand-attached polymer chains reduces surface rigidity upon which adherent cells are attached. Because these spread cells are in tension, a sudden loss of this tension and traction prompts detachment and rounding. Besides, when temperature is decreased, thermo-responsive polymer chain hydration may extend outwards shielding the peptide from integrin access, resulting in cell detachment from the surface [Mitsuhiro et al. 2008]. Peptide shielding may effectively play an important role in cell detachment as cells are known to form and break integrin-RGD bonds to a large extent during adhesion due to the weak binding energy of these bonds compared to other biological bonds [Ebara et al. 2008].

Concerning cell detachment kinetics (Fig. 5), one can observe a two-step process independently on the incubation temperature. A fast cell detachment rate was achieved in the first step which corresponds to the initial 60 minutes. This clearly implies that temperature-induced T24-RGD hydration is a rapid process whose onset takes place before the first 30 minutes. The second step is best described by a remarkable change in the slope reflecting a reduction on cell detachment rate. Indeed, cell detachment continued slowly onward and was not finished even after a 240-min incubation. This result well correlates with data on cell release kinetics from RGD-grafted pNIPAM [Mitsuhiro et al. 2008].

Considering cell incubation at 28 and 20°C, detachment rate was improved as temperature was reduced.

About 30 and 70% of cell detachment, respectively, was observed after 60 min. However, at the lowest temperature (4°C), the number of detached cells was smaller than at 20°C for both 30, 60, 120 and 240-min incubation times. Such result showed that cell detachment was not directly correlated with reduced temperature and seems a controversial observation. As the chains of T24-RGD are assumed to be hydrated and maintain expanded conformations at lower temperatures resulting in reduced interactions between the cells and polymer surfaces, we should rather expect an increased detachment rate as temperature is reduced. That fact it is not observed experimentally implies that the hydration of T24-RGD may not completely explain cell detachment from culture surfaces but might also be related to modified cell metabolism and membrane morphology at very low tempratures.

In order to check the stability of cell metabolic activity over passages, temperature-assisted method was used to detach A375 cells on T24-RGD coated wells while trypsinization was carried out for uncoated standard wells during 3 weeks (cell replating once a week). A third group consisted of A375 cells that underwent one passage from T24-RGD coated wells using the temperature-assisted method for cell detachment and two passages from standard uncoated wells using trypsinization for cell detachment. A375 cells on T24-coated wells could not be passaged as the amount of obtained cells was inferior to 20 000 cells per well. After the third culture week, 20 000 cells from T24-RGD coated wells and uncoated standard wells were replated on uncoated standard wells and incubated overnight. Their metabolic activity was evaluated via a MTT test (Fig. 6). Obtained data indicated that cells cultured on T24-RGD supported similar levels of cell metabolic activity when compared with uncoated standard wells (P = 0.7909). The same is true (P = 0.3711) for the cells transferred from T24-RGD (1-week culture) to uncoated standard wells (two-week culture). This implies that neither the polymer itself nor the temperature-assisted method alter cell metabolic activity. Two additional experiments were carried out to confirm this hypothesis.

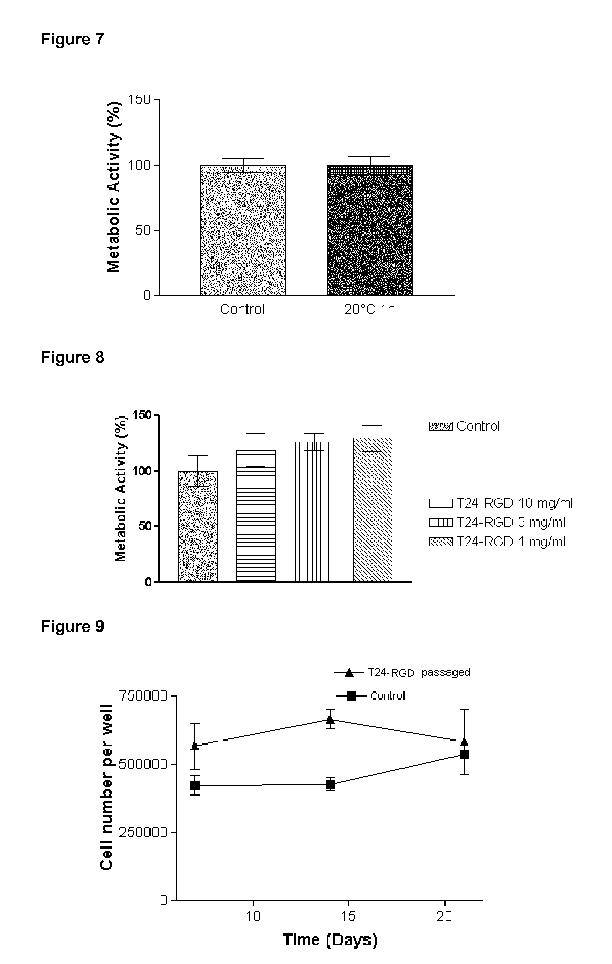

First, a MTT test was performed for A375 cells submitted to the temperature-assisted method for cell detachment. This test was performed on cell plated on standard uncoated wells to set aside the effect of polymer exposure itself. It was observed that the temperature-assisted method for cell detachment did not change cell metabolism. There was no statistically significant difference (P = 0.9799) concerning the metabolic activity between treated and untreated control cells (Fig. 7).

Another MTT test was performed for A375 cells exposed to T24-RGD in solution during two days in order to verify the influence of the polymer itself on the cell metabolic activity (Fig. 8). We tested three concentrations: 1, 5 and 10 mg/mL of T24-RGD. This choice was based on a test reported in the literature to evaluate the effect of other thermo-responsive polymers on cell metabolic activity. It was found that the exposure of A375 cells to 1, 5 or 10 mg/mL of T24-RGD did not reduce the metabolic activity compared to control the group (P values of 0.0667; 0.0630 and 0.2041, respectively). The obtained values were at or above 100% as compared to control. Regarding literature data [Vihola et al. 2005], T24-RGD can be considered as well tolerated as other thermo-responsive polymers such as poly(N-isopropylacrylamide), poly(N-vinylcaprolactam) and amphiphilic modified poly(N-vinylcaprolactam).

The lack of effect related to the polymer exposure or to the temperature-assisted method on cell metabolic activity is consistent with the results presented on Fig 6. As both are well tolerated, it is reasonable to expect a full metabolic activity for cells cultured on T24-RGD during three weeks, passaged three times by the temperature-assisted method.

When A375 cells were passaged from T24-RGD coated wells to uncoated standard wells, their proliferation rate was similar to that observed for cells cultured on uncoated standard wells (P = 0.0604) (Fig. 9).

In overall, these results indicate that functionalized xyloglucan-based films are of great interest for the culture of adherent cells and for their protease-free detachment.

The invention thus provides thermoresponsive films allowing cell adhesion and proliferation while enabling temperature-assisted cell detachment of gret potential useful for the culture of numerous kinds of cells, in particular of stem cells.

### References

Andriola Silva Brun-Graeppi, A.K., Richard, C. Bessodes, M. Scherman, D. Narita, T. Ducouret, G.et al., Study on the sol-gel transition of xyloglucan hydrogels. Carbohydrate Polymers. 2010;80:555-562.
Andriola Silva Brun-Graeppi, A.K., Richard, C. Bessodes, M. Scherman, D. Narita, T. Ducouret, G. et al., The effect of sterilization methods on the thermogelation properties of xyloglucan hydrogels. Polymer Degradation and Stability. 2010;95:254-259.
Ebara, M., M. Yamato, T. Aoyagi, A. Kikuchi, K. Sakai, and T. Okano, The effect of extensible PEG tethers on shielding between grafted thermo-responsive polymer chains and integrin-RGD binding. Biomaterials. 2008;29:3650-3655.
Joannidesa, A., C. Fiore-Hricha, K. Westmorea, M. Caldwella, A. Compstona, N. Allenb, et al., Automated mechanical passaging: a novel and efficient method for human embryonic stem cell expansion. Stem Cells. 2006;24:230-235.
Marre, A.D., H. Soyez, D. Permentier, and E. Schacht, 4-Nitrophenyl chloroformate activation of poly-a, b-(N-(2-hydroxyethyl)-D, L-aspartamide) and poly-a, b-(N-(2, 3-dihydroxypropyl)-D, L-aspartamide). Polymer-Letchworth. 1995;36:4031-4038.
Merten, O.-W. Cell Detachment. In: "Encyclopedia of Industrial Biotechnology: Bioprocess, Bioseparation, and Cell Technology", ed Flickinger M.C., J. Wiley & Sons Ltd., New York/USA, 2010; pp. 1-22.
Mitsuhiro, E., Y. Masayuki, A. Takao, K. Akihiko, S. Kiyotaka, and O. Teruo, A novel approach to observing synergy effects of PHSRN on integrin-RGD binding using intelligent surfaces. Advanced Materials. 2008;20:3034-3038.
Ohya, S., S. Kidoaki, and T. Matsuda, Poly(N-isopropylacrylamide) (PNIPAM)-grafted gelatin hydrogel surfaces: interrelationship between microscopic structure and mechanical property of surface regions and cell adhesiveness. Biomaterials. 2005;26:3105-3111.
Seo, S.J., I.K. Park, M.K. Yoo, M. Shirakawa, T. Akaike, and C.S. Cho, Xyloglucan as a synthetic extracellular matrix for hepatocyte attachment. Journal of Biomaterials Science, Polymer Edition. 2004;5:1375-1387.
Shirakawa, M., K. Yamatoya, and K. Nishinari, Tailoring of xyloglucan properties using an enzyme. Food Hydrocolloids. 1998;12:25-28.
Vihola, H., A. Laukkanen, L. Valtola, H. Tenhu, and J. Hirvonen, Cytotoxicity of thermosensitive polymers poly (N-isopropylacrylamide), poly (N-vinylcaprolactam) and amphiphilically modified poly (N-vinylcaprolactam). Biomaterials. 2005;26:3055-3064.
Zhang, J.L., Srivastava, R.S. and Misra, R.D.K. Core-shell magnetite nanoparticles surface encapsulated with smart stimuli-responsive polymer: synthesis, characterization, and LCST of viable drug-targeting delivery system. Langmuir. 2007;23:6342-6351.

## Claims

1. Cell culture support comprising a functionalized xyloglucan hydrogel-based film, such as produced by
. removal of at least 35%, preferably from 35-45%, of the galactose groups of the xyloglucan residue,
. activation of part of the remaining free -OH groups of the sugar residues of the modified xyloglucan (galactose, xylose)
for grafting a bioadhesive peptide as ligand or containing the specific motifs of ligand specific for the attachment site of cell surface proteins.

2. The culture support of claim 1, wherein said partial galactose removal step is carried out by reacting the xyloglucane with β-galactosidase resulting in a partially degalactosylated xyloglucane.

3. The culture support of claim 1 or 2, wherein the activation step comprises reacting at least some of the remaining groups of the partially degalactosylated xyloglucane with an agent such as 4-nitrophenyl chloroformate.

4. The culture support of anyone of claims 1-3, wherein the activated partially degalactosylated xyloglucane is reacted with a peptide containing the cell attachment site of surface proteins, particularly a sequence part of an integrin-ligand.

5. The culture support of claim 4, wherein the peptide contains the sequence RGD.

6. The culture support of claim 5 wherein peptides containing said sequence comprise part of the ones from fibronectin, vitronectin, fibrinogen, von Willebrand factor, thrombospondin, laminin, entactin, tenascin, osteopontin, bone sialoprotein.

7. The culture support according to claim 4, wherein the peptide is CGGNGEPRGDTYRAY.

8. The culture support of claim 4, wherein the peptide is selected in the group comprising the cyclic peptide DfKRG, KKQRFRHRNRKG from vitronectin, CDPGYIGSR, IKVAV, RNIAEIIKDI, YFQRYLI, PDSRG, LNIVSVNGRHX and DNRIRLQAKXX from laminin, *DGEA and* GVKGDKGNPGWPGAE peptide sequences from collagen, VAPG sequence of elastin, SIGFRGDGQTC from entactin and FHRRIKA from bone sialoprotein.

9. The culture support of anyone of claims 1-8, wherein the coated support is incubated with the cell culture medium for conditioning.

10. The culture support of anyone of claims 1-9, comprising an hydrogel of more than 2% of total xyloglucan residue, advantageously of about 4%.

11. The culture support of anyone of claims 1-9, comprising an hydrogel containing 3% at least, preferably around 5%, of peptide-grafted partially degalactosylated xyloglucan.

12. The culture support of anyone of claims 1-8, comprising a mixture of said hydrogel at 3% at least, preferably around 5%, with a partially degalactosylated non grafted xyloglucan used at about 2-4%, preferably 3%.

13. The culture support of claim 12, comprising the grafted xyloglucan and non grafted xyloglucan in a ratio of 1:1vol/vol.

14. The culture support of anyone of claims 1-13, wherein a spacer, such as PEG is used to enhance the interaction between the peptide and the cell.

15. A method for preparing a culture support according to anyone of claims 1-14 comprising drying the hydrogel to generate a film.

16. A method for the detachment of adherent cells from a support according to anyone of claims 1-15, performed by temperature reduction (temperature-assisted).

17. The method of claim 16, wherein the detachment occurs at the ambient temperature.

18. The method of claim 16, wherein the detachment is obtained by reducing the temperature, for example by addition of fresh culture medium, followed by incubation at the ambient temperature.
